# EUROPÄISCHE PATENTANMELDUNG

(11) **EP 1 818 330 A1**
(43) Veröffentlichungstag der Anmeldung: **15.08.2007**
(21) Anmeldenummer: 06101653.1
(22) Anmeldetag: 14.02.2006
(51) Int. Cl.: C07D 401/12, A61K 31/55, A61P 7/02

(54) **Substituierte Prolinamide, deren Herstellung und deren Verwendung als Arzneimittel**

(71) Anmelder: Boehringer Ingelheim Pharma GmbH & Co.KG, 55216 Ingelheim am Rhein (DE)
(72) Erfinder: Die Erfindernennung liegt noch nicht vor

(57) **Zusammenfassung**

Gegenstand der vorliegenden Erfindung sind neue substituierte Prolinamide der allgemeinen Formel (I) 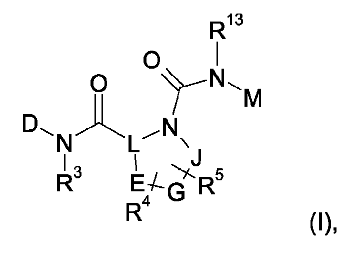 in der D, L, E, G, J, M, R³, R⁴, R⁵ und R¹³ wie in Anspruch 1 definiert sind, deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

## Beschreibung

Gegenstand der vorliegenden Erfindung sind neue substituierte Prolinamide der allgemeinen Formel (I) 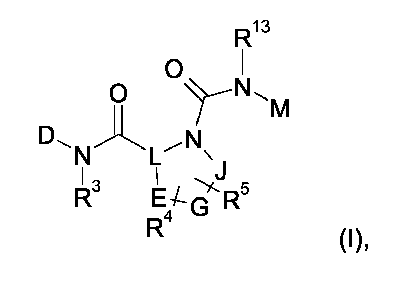 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel (I) sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

Gegenstand der vorliegenden Anmeldung sind neue Verbindungen der obigen allgemeinen Formel (I), deren Herstellung, die die pharmakologisch wirksamen Verbindungen enthaltenden Arzneimittel, deren Herstellung und Verwendung.

Eine 1. Ausführungsform der vorliegenden Erfindung umfasst diejenigen Verbindungen der allgemeinen Formel (I), in denen
- D: ein substituiertes bicyclisches Ringsystem der Formel (II) 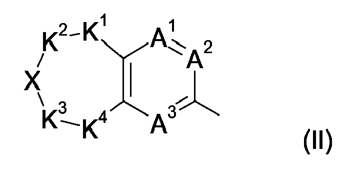 darstellt, in dem
K¹ und K⁴ jeweils unabhängig voneinander eine -CH₂-, -CHR^{7a}-, -CR^{7b}R^{7c}- oder eine -C(O)-Gruppe bedeuten, und wobei
R^{7a}/R^{7b}/R^{7c} jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe,
eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7a}/R^{7b}/R^{7c} zusammen mit dem Ringkohlenstoffatom oder den Ringkohlenstoffatomen einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon- oder [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem N-Atom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³ jeweils unabhängig voneinander eine -CH₂-, -CHR^{8a}-, -CR^{8b}R^{8c}- oder eine -C(O)-Gruppe bedeuten, wobei
R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem Stickstoffatom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl-sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonyl-gruppe substituiert sein können, oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
- A¹: entweder N oder CR¹⁰ bedeutet,
- A²: entweder N oder CR¹¹ bedeutet,
- A³: entweder N oder CR¹² bedeutet, wobei R¹⁰, R¹¹ und R¹² jeweils unabhängig voneinander ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
- -L-E-G-J-: eine -C-C-C-C- oder -C-C=C-C-Gruppe bedeutet, die durch R⁴ und R⁵ substituiert sein kann, und
- R³: ein Wasserstoffatom oder eine C₁₋₃-Alkylgruppe bedeutet, und
- R⁴: ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁-₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₀₋₃-alkyloxy-, Heteroaryl-C₀₋₃-alkyl-oxy-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom- und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet, und
wenn -L-E-G-J- eine -C-C-C-C-Gruppe bedeutet, kann R⁴ an E oder G auch ein Fluoratom oder eine Hydroxy-, Methoxy-, C₂₋₅-Alkenyl-oxy-, C₂₋₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₃Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder-S-O-Bindung bilden, ausgeschlossen ist, und
- R⁵: ein Wasserstoffatom oder eine C₁₋₅Alkylgruppe bedeutet, oder sofern R⁵ mit E oder G verknüpft ist auch eine Hydroxy- oder Methoxy-gruppe darstellen kann, oder
- R⁴ und R⁵: zusammen mit dem gleichen Kohlenstoffatom
eine -C(O)- Gruppe,oder eine -C(F₂)- Gruppe bilden können, oder
- R⁴ und R⁵: zusammen mit dem gleichen Kohlenstoffatom oder zwei benachbarten Kohlenstoffatomen, an denen sie gebunden sind,
eine C₃₋₇-Cycloalkyl- oder C₅₋₇-Cycloalkenylgruppe bilden können,
wobei eine der Methylengruppen einer C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen einer C₄₋₇-Cycloalkylgruppe zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei 1 bis 3 Kohlenstoffatome einer C₃₋₇-Cycloalkylgruppe gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass eine solche, zusammen aus R⁴ und R⁵ gebildete C₃₋₇-Cycloalkylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
- R¹³: ein Wasserstoffatom oder eine C₁₋₅Alkylgruppe bedeutet,
- M: einen gegebenenfalls durch R² und R⁶ substituierten Phenyl- oder Pyridylring bedeutet, in dem
R² ein Fluor-, Chlor-, Brom- oder Jod-atom oder eine Methyl-, Ethyl-, Vinyl-, Methoxy-, Ethinyl-, Cyano- oder -C(O)NH₂-Gruppe darstellt, und
R² xein Wasserstoff-, Fluor-, Chlor-, Brom- oder Jod-atom oder eine Hydroxy-, Methoxy-, Trifluormethoxy-, eine gegebenenfalls durch Fluoratome substituierte C₁₋₃-Alkyl-, Cyano-, Amino-, oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Beispiele für monocyclische Heteroarylgruppen sind die Pyridyl-, *N*-Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thienyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[*c*]furanyl-, Benzothiophenyl-, Benzo[*c*]thiophenyl-, Benzothiazolyl-, Benzo[*c*]isothiazolyl-, Benzo[*d*]isothiazolyl-, Benzooxazolyl-, Benzo[*c*]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]triazinyl-, Benzo[1,2,4]triazinyl-, Benzotriazolyl-, Cinnolinyl-, Chinolinyl-, *N*-Oxy-chinolinyl-, Isochinolinyl-, Chinazolinyl-, *N*-Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylgruppen sind die Methyl-, Ethyl-, 1-Propyl-, 2-Propyl-, *n*-Butyl-, *sec*-Butyl-, *tert*-Butyl-, 1-Pentyl-, 2-Pentyl-, 3-Pentyl-, *neo*-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxy-, *n*-Butyloxy-, *sec*-Butyloxy-, *tert*-Butyloxy-, 1-Pentyloxy-, 2-Pentyloxy-, 3-Pentyloxy- oder *neo*-Pentyloxy-Gruppe.

Beispiele für die voranstehend in den Definitionen erwähnten C₂₋₅-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl- oder 2-Ethyl-prop-2-en-1-yl -Gruppe,

Beispiele für die voranstehend in den Definitionen erwähnten C₂₋₅-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl- oder 3-Methyl-1-butin-3-yl-Gruppe.

Eine 2. Ausführungsform der vorliegenden Erfindung umfasst diejenigen Verbindungen der allgemeinen Formel (I), in denen E, G, J, L, M, R³-R⁵ und R¹³ wie in Ausführungsform 1 beschrieben definiert sind und
- D: ein substituiertes bicyclisches Ringsystem der Formel (II) 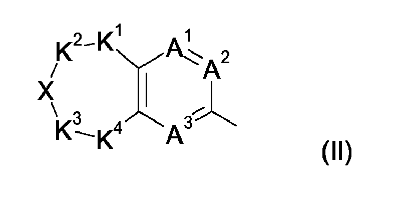 darstellt, in dem
K¹ und K⁴ jeweils unabhängig voneinander eine -CH₂-, -CHR^{7a}-, -CR^{7b}R^{7c}- oder eine -C(O)-Gruppe bedeuten, und wobei
R^{7a}/R^{7b}/R^{7c} jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe,
K² und K³ jeweils unabhängig voneinander eine -CH₂-, -CHR^{8a}-, -CR^{8b}R^{8c}- oder eine -C(O)- Gruppe bedeuten, wobei
R^{8a}/R^{8b}/R^{8c} jeweils unabhängig voneinander eine C₁₋₅-Alkylgruppe bedeutet, und insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
- A¹: entweder N oder CR¹⁰ bedeutet,
- A²: entweder N oder CR¹¹ bedeutet,
- A³: entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten.

Eine 3. Ausführungsform der vorliegenden Erfindung umfasst diejenigen Verbindungen der Ausführungsformen 1 oder 2, in denen
- X: eine NR¹-Gruppe bedeutet, in der
R¹ ein Wasserstoffatom oder eine C₁₋₅-Alkyl-, Allyl- oder Cyclopropylgruppe bedeutet, und
- A¹: CR¹⁰ bedeutet,
- A²: CR¹¹ bedeutet,
- A³: CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten.

Eine 4. Ausführungsform der vorliegenden Erfindung umfasst diejenigen Verbindungen der allgemeinen Formel (I) in denen D, E, G, J, L, M, R³ und R¹³ wie in Ausführungsform 1, 2 oder 3 beschrieben definiert sind, und in der
- R⁴: ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
wenn -L-E-G-J- eine -C-C-C-C-Gruppe bedeutet, kann R⁴ an E oder G auch ein Fluoratom oder eine Hydroxy-, Methoxy-, C₂₋₅-Alkenyl-oxy-, C₂₋₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₃Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder-S-O-Bindung bilden, ausgeschlossen ist, und
- R⁵: ein Wasserstoffatom oder eine C₁₋₅Alkylgruppe bedeutet, oder sofern R⁵ mit E oder G verknüpft ist auch eine Hydroxy- oder Methoxy-gruppe darstellen kann oder
- R⁴ und R⁵: zusammen mit dem gleichen Kohlenstoffatom
eine -C(O)- Gruppe,oder eine -C(F₂)- Gruppe bilden können, oder
- R⁴ und R⁵: zusammen mit dem gleichen Kohlenstoffatom oder zwei benachbarten Kohlenstoffatomen, an denen sie gebunden sind,
eine C₃₋₇-Cycloalkyl-gruppe bilden können,
wobei eine der Methylengruppen einer C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen einer C₄₋₇-Cycloalkylgruppe zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können.

Eine 5. Ausführungsform der vorliegenden Erfindung umfasst diejenigen Verbindungen der Ausführungsformen 1, 2, 3 oder 4, in denen
- -L-E-G-J-: eine -C-C-C-C-Gruppe bedeutet, die durch R⁴ und R⁵, die wie oben in den Ausführungsformen 1, 2, 3 oder 4 definiert sind, substituiert sein kann.

Eine 6. Ausführungsform der vorliegenden Erfindung umfasst diejenigen Verbindungen der Ausführungsformen 1, 2, 3, 4 oder 5, in denen
- D: einen substituierten Benzazepinylrest der Formel (IIa) 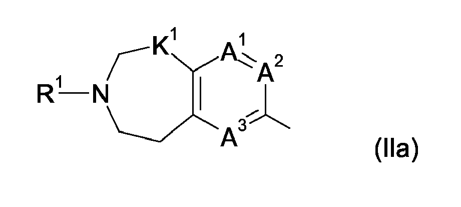 darstellt, in dem
K¹ eine -CH₂-, -CHR^{7a}-, -CR^{7b}R^{7c}- oder eine -C(O)-Gruppe bedeutet, wobei
R^{7a} eine C₁₋₅-Alkylgruppe bedeutet und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können, und
R¹ ein Wasserstoffatom oder eine C₁₋₅-Alkyl-, Allyl- oder
Cyclopropylgruppe bedeutet, und in denen
A¹ CR¹⁰ bedeutet,
A² CR¹¹ bedeutet,
A³ CR¹² bedeutet, wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
- -L-E-G-J-: eine -C-C-C-C-Gruppe bedeutet, die durch R⁴ und R⁵ substituiert sein kann, und
- R³: ein Wasserstoffatom bedeutet, und
- R⁴: ein Wasserstoffatom oder
eine geradkettige oder verzweigte C₁₋₃-Alkylgruppe bedeutet, wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe bedeutet, oder
wenn R⁴ an E oder G angebunden ist auch ein Fluoratom oder eine Hydroxy-, Methoxy-, C₂₋₅-Alkenyl-oxy-, C₁₋₅-Alkyl-oxy-, C₃₋₆-Cycloalkyloxy-, C₁₋₅-Alkylaminocarbonyloxy-, Di(C₁₋₅-alkyl)aminocarbonyloxy- oder C₄₋₇-Cycloalkyleniminocarbonyloxy-gruppe darstellen kann,
- R⁵: ein Wasserstoffatom oder eine C₁₋₅Alkylgruppe bedeutet, oder sofern R⁵ mit E oder G verknüpft ist auch eine Hydroxy- oder Methoxy-gruppe darstellen kann, oder
- R⁴ und R⁵: zusammen mit dem Kohlenstoffatom an dem sie gebunden sind eine C=O- oder eine -CF₂-Gruppe bedeuten können, und
- R¹³: ein Wasserstoffatom bedeutet,
- M: einen durch R² in 4-Position substituierten Phenyl- oder durch R² in 5-Position substituierten Pyridylring bedeutet, in dem
R² ein Fluor-, Chlor-, Bromatom, eine Methoxy- oder Ethinyl-Gruppe darstellt, und
- R⁶: ein Wasserstoffatom darstellt.

Erfindungsgemäß erhält man die Verbindungen der allgemeinen Formel (I) nach an sich bekannten Verfahren, beispielsweise nach folgenden Verfahren:
(a) Die Herstellung einer Verbindung der allgemeinen Formel (III) 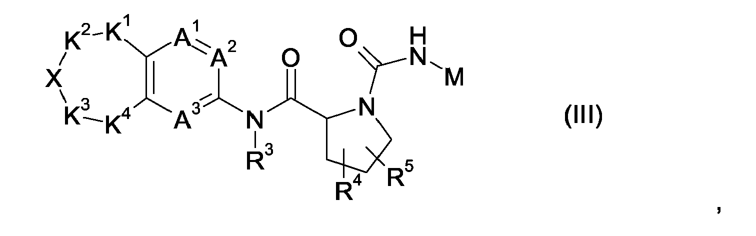 in der A¹ bis A³, K¹ bis K⁴, M und R¹ bis R⁶ wie in Ausführungsform 1 erwähnt definiert sind,
   und die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein kann, und deren Schutzgruppen in Literatur nach bekannter Methode abgespalten werden können,
   wird in den Ausführungsbeispielen beschrieben oder kann beispielsweise nach einem der folgenden Formelschemata 1 und 2 oder in Analogie zu den Syntheseverfahren die in WO2004/87695, WO2004/87646 oder in WO2003/45912 beschrieben sind durchgeführt werden. 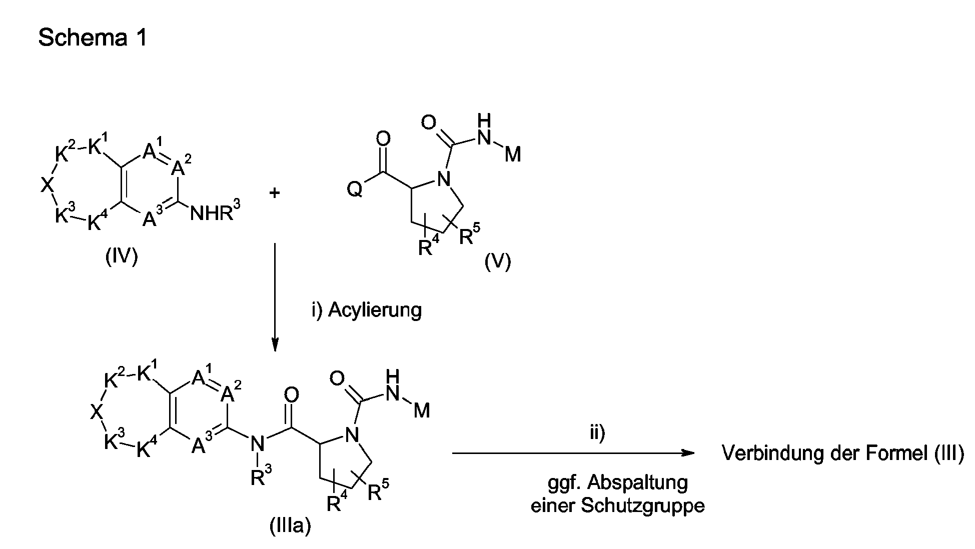 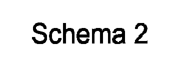 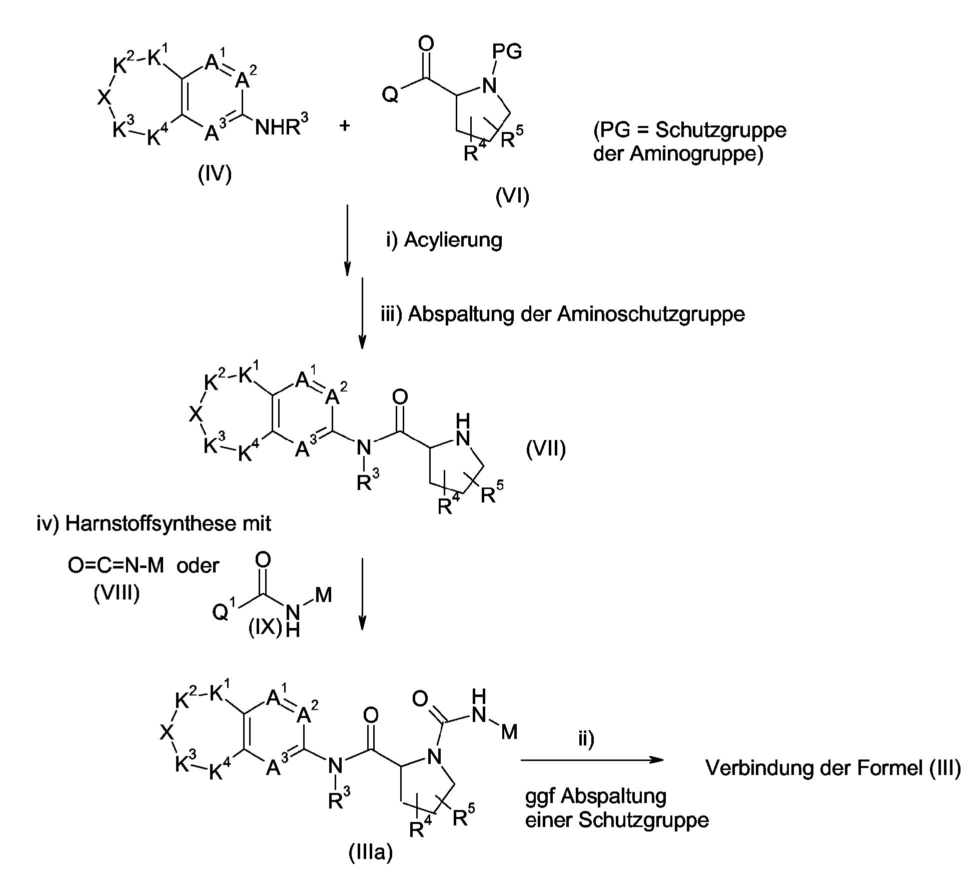 wobei
   Q/Q¹ eine Austrittsgruppe oder eine *in-situ* in eine Austrittsgruppe überführbare Gruppe wie beispielsweise ein Halogenatom, eine Hydroxy-, C₁₋₄-Alkyloxy-, Alkyloxycarbonyloxy-, 4-Nitrophenyloxy-, eine Trichlormethyl- oder Acyloxy-gruppe darstellt, und PG eine in Literatur bekannte Schutzgruppe der Aminofunktion wie beispielsweise eine tert.-Butoxycarbonyl-, Benzyloxycarbonyl- oder eine Trifluoracetyl-gruppe darstellt.
   Die in Schema 1 und 2 beschriebenen Reaktionsstufen i) -iv) können auf die in den Beispielen beschriebene Weise oder nach in Literatur bekannter Bedingungen beispielsweise wie folgt durchgeführt werden:
   i) Acylierung eines Amins (IV) mit einer gegebenenfalls aktivierten Carbonsäure (V) oder (VI) :
      Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Dimethylsulfoxid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 100°C, durchgeführt.
      Die Acylierung kann jedoch auch mit der freien Säure gegebenenfalls in Gegenwart eines die Säure aktivierenden Mittels oder eines wasserentziehenden Mittels, beispielsweise in Gegenwart von Ethyl-1-ethoxy-1,2-dihydrochinolin-1-carboxylat, Chlorameisensäureisobutylester, Thionylchlorid, Trimethylchlorsilan, Chlorwasserstoff, Schwefelsäure, Methansulfonsäure, *p*-Toluolsulfonsäure, Phosphortrichlorid, Phosphorpentoxid, *N*,*N*'-Dicyclohexylcarbodiimid, *N*,*N*'-Dicyclohexylcarbodiimid/Camphersulfonsäure, *N*,*N*'-Dicyclohexylcarbodiimid/*N*-Hydroxysuccinimid oder 1-Hydroxy-benztriazol, *N*,*N*'-Carbonyldiimidazol, *O*-(Benzotriazol-1-yl)-*N*,*N*,*N'*,*N*'-tetramethyluroniumtetrafluorborat/*N*-Methylmorpholin, *O*-(Benzotriazol-1-yl)-*N*,*N*,*N'*,*N*'-tetramethyl-uroniumtetrafluorborat/*N-*Ethyldüsopropylamin, *O*-Pentafluorophenyl-*N*,*N*,*N*',*N*'-tetramethyluronium-hexafluorophosphat/Triethylamin, *N*,*N*'-Thionyldiimidazol oder Triphenylphosphin/Tetrachlorkohlenstoff, gegebenenfalls unter Zusatz einer Hilfsbase wie Natronlauge, Cäsium-, Kalium- oder Natrium-carbonat oder -hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
      Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995, oder auch im Houben-Weyl Ergänzungsband 22, Thieme Verlag, 2003 und der dort zitierten Literatur beschrieben.
   ii) bzw. iii) Abspaltung einer Schutzgruppe
      Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.
      Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Tetrahydrofuran, Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.
      Die Abspaltung ener Schutzgruppe kann aber auch nach den in T.W. Greene, P.G.M. Wuts in "Protective Groups in Organic Synthesis", Wiley, 1991 und 1999, beschriebenen Verfahren durchgeführt werden.
   iv) Synthese eines Harnstoffs
      Die Umsetzung eines Derivates VII mit einem Isocyanat VIII oder einer gegebenenfalls aktivierten Carbaminsäure IX erfolgt in einem Lösungsmittel wie beispielsweise Wasser, Methylenchlorid, Chloroform, Tetrachlorkohlenstoff, Ether, Tetrahydrofuran, Dioxan, Benzol, Toluol, Acetonitril, Dimethylformamid, Dimethylsulfoxid oder Sulfolan oder einem Gemisch der genannten Lösungsmittel gegebenenfalls unter Zusatz einer Hilfsbase wie Natronlauge, Cäsium-, Kalium- oder Natrium-carbonat oder -hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
(b) Die Bausteine der allgemeinen Formel 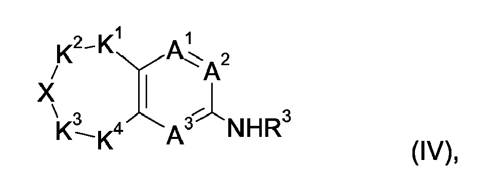 in denen A¹, A², A³, K¹, K², K³, K⁴, X und R³ wie in Ausführungsform 1 erwähnt definiert sind, und
   die gegebenenfalls an vorhandenen Amino-, Hydroxy-, Carboxy- oder Thiol-gruppen durch gängige Schutzgruppen, wie beispielsweise denen in T.W. Greene, P.G.M. Wuts in "Protective Groups in Organic Synthesis", Wiley, 1991 und 1999, beschriebenen geschützt sein können, und deren Schutzgruppen in Literatur nach bekannter Methode im Verlauf der Synthesesequenz zu Verbindungen der Formel (I) abgespalten werden können,
   sind aus der Literatur bekannt, oder deren Synthese wird in den Ausführungsbeispielen beschrieben, oder sie können beispielsweise nach in Literatur bekannter Syntheseverfahren oder in Analogie zu in Literatur bekannten Syntheseverfahren wie beispielsweise in DE4429079, US4490369, DE3515864, US5175157, DE1921861, WO85/00808 bzw. in G. Bobowski et al., J.Heterocyclic Chem. 16, 1525, 1979 oder in P.D. Johnson et al., Bioorg. Med. Chem. Lett 2003, 4197, beschrieben, hergestellt werden.
   Beispielsweise kann eine Verbindung der allgemeinen Formel (IV), in der R³ ein Wasserstoffatom bedeutet und A1, A2, A3, K1, K2, K3, K4 und X wie in Ausführungsform 1 erwähnt definiert sind durch Reduktion der Nitrogruppe einer Verbindungen der allgemeinen Formel (X) 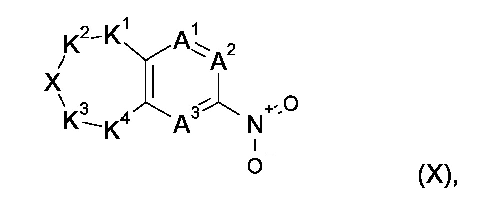 in der A1, A2, A3, K1, K2, K3, K4 und X wie in Ausführungsform 1 erwähnt definiert sind, wie folgt hergestellt werden.
   Die Reduktion der Nitrogruppe wird beispielsweise zweckmäßigerweise in einem Lösungsmittel oder Lösungsmittelgemisch wie Wasser, wäßriger Ammoniumchlorid-Lösung, Salzsäure, Schwefelsäure, Phosphorsäure, Ameisensäure, Essigsäure, Acetanhydrid mit Metallen wie Eisen, Zink, Zinn oder Schwefelverbindungen wie Ammoniumsulfid, Natriumsulfid oder Natriumdithionit oder durch katalytische Hydrierung mit Wasserstoff, beispielsweise unter einem Druck zwischen 0.5 und 100 bar, vorzugsweise jedoch zwischen 1 und 50 bar, oder mit Hydrazin als Reduktionsmittel, zweckmäßigerweise in Gegenwart eines Katalysators wie beispielsweise Raney-Nickel, Palladiumkohle, Platinoxid, Platin auf Mineralfaser oder Rhodium, oder mit komplexen Hydriden wie Lithiumaluminiumhydrid, Natriumborhydrid, Natriumcyanborhydrid, Diisobutylaluminiumhydrid, zweckmäßigerweise in einem Lösungsmittel oder Lösungsmittelgemisch wie Wasser, Methanol, Ethanol, Isopropanol, Pentan, Hexan, Cyclohexan, Heptan, Benzol, Toluol, Xylol, Ethylacetat, Methylpropionat, Glykol, Glykoldimethylether, Diethylenglykoldimethylether, Dioxan, Tetrahydrofuran, *N*-Methylpyrrolidinon, oder aber *N*-Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin, beispielsweise bei Temperaturen zwischen -30 und 250°C, vorzugsweise jedoch zwischen 0 und 150°C, durchgeführt.
(c) Die Bausteine der allgemeinen Formel 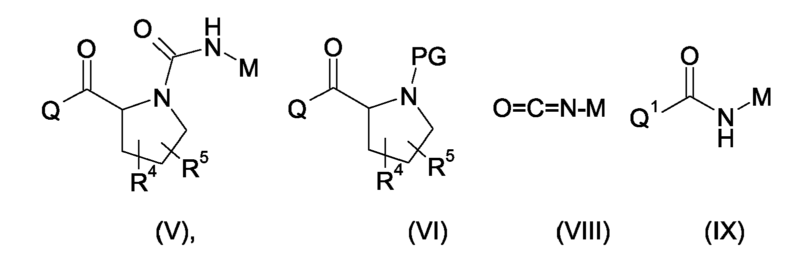 in denen R⁴, R⁵, R⁶ und R² wie in Ausführungsform 1 erwähnt definiert sind, und wobei
   Q/Q¹ beispielsweise eine Hydroxy- oder C₁₋₄-Alkyloxygruppe, ein Halogenatom, eine Alkyloxycarbonyloxy- oder Acyloxygruppe darstellt die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein können und deren Schutzgruppen in literaturbekannterweise im Verlauf der Synthesesequenz zu Verbindungen der Formel (I) abgespalten werden können,
   sind in der Literatur bekannt, oder deren Synthese wird in den Ausführungsbeispielen beschrieben, oder sie können beispielsweise nach in der Literatur bekannten Syntheseverfahren oder in Analogie zu in der Literatur bekannten Syntheseverfahren wie beispielsweise in WO2004/87646 oder W02003/45912 beschrieben, hergestellt werden.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, *tert*.-Butyl-, Trityl-, Benzyl- oder Tetrahydropyranyl-gruppe in Betracht.

Beispielsweise kommt als Schutzrest für eine Carboxylgruppe die Trimethylsilyl-, Methyl-, Ethyl-, *tert*.-Butyl-, Benzyl- oder Tetrahydropyranyl-gruppe in Betracht.

Beispielsweise kommt als Schutzrest für eine Amino-, Alkylamino- oder Iminogruppe die Acetyl-, Trifluoracetyl-, Benzoyl-, Ethoxycarbonyl-, *tert*.-Butoxycarbonyl-, Benzyloxycarbonyl-, Benzyl-, Methoxybenzyl- oder 2,4-Dimethoxybenzyl-gruppe, und für die Aminogruppe zusätzlich die Phthalylgruppe in Betracht.

Beispielsweise kommt als Schutzrest für eine Ethinylgruppe die Trimethylsilyl-, Diphenylmethylsilyl-, tert.Butyldimethylsilyl- oder eine 1-Hydroxy-1-methyl-ethylgruppe in Betracht.

Weitere Schutzgruppen die eingesetzt werden können und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel (I) in ihre Enantiomeren und/oder Diastereomeren aufgetrennt werden.

So lassen sich beispielsweise die erhaltenen Verbindungen der allgemeinen Formel (I), welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel (I) mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch chromatographische Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel (I), falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel (I) sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor VIIa, Faktor IX, Faktor XI und Faktor XII.

Die im Experimentellen Teil angeführten Verbindungen können auf ihre Wirkung auf die Hemmung des Faktors Xa wie folgt untersucht werden.

### Methodik

Enzymkinetische Messung mit chromogenem Substrat. Die durch humanen Faktor Xa aus dem farblosen chromogenen Substrat freigesetzte Menge an p-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material

Tris(hydroxymethyl)-aminomethan-Puffer (100 mMol) und Natriumchlorid (150 mMol), pH 8.0 plus 1 mg/ml Human Albumin Fraction V, Proteasefrei.

Faktor Xa (Calbiochem), Spez. Aktivität: 217 IU/mg, Endkonzentration: 7 IU/ml pro Reaktionsansatz.

Substrat S 2765 (Chromogenix), Endkonzentration: 0.3 mM/I (1 KM) pro Reaktionsansatz.

Testsubstanz: Endkonzentration 100, 30, 10, 3, 1, 0.3, 0.1, 0.03, 0.01, 0.003, 0.001 µmol/l.

### Durchführung

10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigen IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulcerativen Colitis, der Prophylaxe und Behandlung der Koronarthrombose, der Prophylaxe des Schlaganfalls und der Verhinderung der Occlusion von Shunts.

Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von Fibrin-abhängigen Gewebsadhäsionen und/oder Narbengewebebildung sowie zur Förderung von Wundheilungsprozessen geeignet.

Aufgrund ihrer pharmakologischen Eigenschaften eignen sich außerdem die neuen Verbindungen und deren physiologisch verträgliche Salze zur Behandlung der Alzheimer- und Parkinson'schen Krankheit. Eine Rationale dafür ergibt sich zum Beispiel aus folgende Befunden, aus denen man schließen kann, dass Thrombinhemmer bzw. Faktor Xa Hemmer, durch Hemmung der Thrombinbildung bzw. -aktivität, wertvolle Medikamente in der Behandlung der Alzheimer- und Parkinson'schen Krankheit darstellen könnten. Klinische und experimentelle Studien legen nahe, dass neurotoxische Mechanismen, beispielsweise die mit der Aktivierung von Proteasen der Gerinnungskaskade einhergehende Entzündung, beteiligt ist am Absterben von Neuronen infolge von Hirntraumata. Verschiedene Studien deuten auf eine Beteiligung von Thrombin bei neurodegenerativen Prozessen hin, beispielsweise infolge eines Schlaganfalls, wiederholter Bypassoperation oder traumatischen Hirnverletzungen. Eine erhöhte Thrombinaktivität konnte beispielsweise noch Tage nach peripherer Nervenverletzung nachgewiesen werden. Es konnte weiterhin gezeigt werden, dass Thrombin eine Neuritenretraktion, sowie Glia-Proliferation, und Apoptose in Primärkulturen von Neuronen und Neuroblastomzellen hervorruft (zur Übersicht siehe: Neurobiol. Aging, 2004, 25(6), 783-793). Darüberhinaus deuten verschiedene in vitro Studien an Gehirnen von Patienten mit Alzheimer-Krankheit daruf hin, dass Thrombin in der Pathogenese dieser Krankheit eine Rolle spielt (Neurosci. Lett., 1992, 146, 152-54). Eine Anreicherung immunreaktiven Thrombins konnte in Neuriten-Plaques in Gehirnen von Alzheimer-Patienten nachgewiesen werden. In vitro wurde gezeigt, dass Thrombin ebenfalls eine Rolle bei der Regulation und Stimulation der Produktion des "Amyloid Precursor Proteins" (APP) spielt sowie bei der Spaltung des APP in Fragmente, welche in den Amyloid-Plaques im Gehirn von Alzheimer-Patienten nachgewiesen werden können. Weiterhin konnte gezeigt werden, dass die thrombin-induzierte mikrogliale Aktivierung in vivo zur Degeneration von nigralen dopaminergen Neuronen führt. Diese Befunde lassen den Schluss zu, dass mikrogliale Aktivierung -ausgelöst durch endogene Substanz(en) wie beispielsweise Thrombin- beteiligt sind am neuropathologischen Prozess des Zelltodes dopaminerger Neurone, wie er bei Patienten mit Parkinson'scher Krankheit vorkommt (J. Neurosci., 2003, 23, 5877-86).

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel (I), gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

### Experimenteller Teil

Die nachfolgenden Beispiele sollen die Erfindung näher erläutern, ohne diese jedoch in ihrem Umfang zu beschränken.

Für die hergestellten Verbindungen liegen in der Regel Schmelzpunkte und/oder IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX*^{™}*, 35-70 µm) verwendet. Falls nähere Angaben zur Konfiguration fehlen, bleibt offen, ob es sich um reine Stereoisomere oder um Enantiomeren-/Diastereomerengemische handelt.

Die HPLC-MS Daten wurden unter den folgenden Bedingungen erzeugt.

Waters Alliance 2690, Waters ZQ2000 Massen Spektrometer mit Diodenarraydetektor 996.
Als mobile Phase wurde eingesetzt:
A: Wasser mit 0.10% TFA
B: Acetonitril mit 0.8% TFA

| Zeit in min | %A | %B | Flussrate in ml/min |
|---|---|---|---|
| 0.00 | 95 | 5 | 1.00 |
| 0.10 | 95 | 5 | 1.00 |
| 3.10 | 2 | 98 | 1.00 |
| 4.50 | 2 | 98 | 1.00 |
| 5.00 | 95 | 5 | 1.00 |

Als stationäre Phase diente eine Säule X-Terra MS C18, 2.5 µm, 4.6 mm x 30 mm.

Die Diodenarraydetektion erfolgte im Wellenlängenbereich 210-500 nm.

In den Versuchsbeschreibungen werden die folgenden Abkürzungen verwendet.
- DCM: Dichlormethan
- DIPEA: *N*-Ethyl-diisopropylamin
- DMF: *N,N*-Dimethylformamid
- EtOH: Ethanol
- ges.: gesättigt
- h: Stunde(n)
- HATU: *O*-(7-Azabenzotriazol-1-yl)-*N*,*N*,*N*',*N*'-tetramethyluronium-hexafluorphosphat
- i. Vak.: im Vakuum
- konz.: konzentriert
- min: Minute(n)
- NMM: *N*-Methyl-morpholin
- R_{f}: Retentionsfaktor
- Rₜ: Retentionszeit
- TBTU: *O*-(Benzotriazol-1-yl)-*N*,*N*,*N*',*N*'-tetramethyluroniumtetrafluorborat
- TEA: Triethylamin
- TFA: Trifluoressigsäure
- THF: Tetrahydrofuran

### Beispiel 1

### (2R,4R)-4-Methoxy-pyrrolidin-1,2-dicarbonsäure-1-[(4-chlor-phenyl)-amid]-2-(3-methyl-2,3,4,5-tetrahydro-1H-benzo[d]azepin-7-yl)-amid

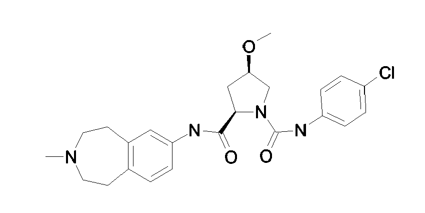

### (a) (2R,4R)-4-Methoxy-pyrrolidin-1,2-dicarbonsäure-1-tert.butoxy-ester-2-(3-methyl-2,3,4,5-tetrahydro-1H-benzo[d]azepin-7-yl)-amid

0.170 g (0.693 mmol) (2R,4R)-4-Methoxy-pyrrolidin-1,2-dicarbonsäure-1-tert.butoxy-ester werden in 5.0 ml THF gelöst, mit 0.19 ml (1.7 mmol) NMM und 0.234 g (0.728 mmol) TBTU verstetzt und 15 min gerührt. Anschließend werden 0.122 g (0.693 mmol) 7-Amino-3-methyl-2,3,4,5-tetrahydro-1*H*-benzo[*d*]azepin zugegeben und 16 h gerührt. Das Reaktionsgemisch wird i.Vak. konzentriert, mit 20 ml Essigester versetzt und sukzessiv mit ges. NaHCO₃-Lösung, ges. NaCl-Lösung und Wasser gewaschen und dann mir Na₂SO₄ und i. Vak. bis zur Trockene eingeengt.
Ausbeute: 0.210 g (75%)
R_{f}-Wert: 0.8 (Kieselgel; Dichlormethan/Ethanol/Ammoniak = 80:20:2)
C₂₂H₃₃N₃O₄ (403.515)
Massenspektrum: (M+H)⁺ = 404

### (b) (2R,4R)-4-Methoxy-pyrrolidin-2-carbonsäure-(3-methyl-2,3,4,5-tetrahydro-1H-benzo[d]azepin-7-yl)-amid

Zu einer Lösung aus 0.210 g (0.520 mmol) (2R,4R)-4-Methoxy-pyrrolidin-1,2-dicarbonsäure-1-tert.butoxy-ester-2-(3-methyl-2,3,4,5-tetrahydro-1*H-*benzo[*d*]azepin-7-yl)-amid in 2.0 ml THF werden über 2 h 1.5 ml 6 M HCl zugegeben und insgesamt 18 h gerührt. Das Reaktionsgemisch wird i. Vak. eingeengt, mehrmals mit Methanol versetzt und erneut eingeengt. Rohausbeute: 0.230 g (quantitativ)
R_{f}-Wert: 0.25 (Kieselgel; Dichlormethan/Ethanol/Ammoniak = 80:20:2)
C₁₇H₂₅N₃O₂ (303.515) x 2 HCl
Massenspektrum: (M+H)⁺ = 304

### (c) (2R,4R)-4-Methoxy-pyrrolidin-1,2-dicarbonsäure-1-[(4-chlor-phenyl)-amid]-2-(3-methyl-2,3,4,5-tetrahydro-1H-benzo[d]azepin-7-yl)-amid

Zu einer Lösung aus 0.100 g (0.266 mmol) (2R,4R)-4-Methoxy-pyrrolidin-2-carbonsäure-(3-methyl-2,3,4,5-tetrahydro-1*H*-benzo[*d*]azepin-7-yl)-amid in 2.0 ml Dioxan und 2.0 ml DMF werden 40.8 mg (0.266 mmol) 4-Chlorphenylisocyanat zugegeben und 18 h gerührt. Das Reaktionsgemisch wird i.Vak. konzentriert und durch präp. HPLC gereinigt (Methode A)
Ausbeute: 15 mg (11%)
R_{f}-Wert: 0.4 (Kieselgel; Dichlormethan/Ethanol/Ammoniak = 80:20:2)
C₂₄H₂₉ClN₄O₃ (456.974) x HCOOH
Massenspektrum: (M+H)⁺ = 457/459 (Chlorisotope)

Analog können die folgenden Verbindungen hergestellt werden:

| **Bsp.** | **Strukturformel** | **Ausbeute** | **Massenpeak(s)** | **DC/HPLC** |
|---|---|---|---|---|
| **2** | 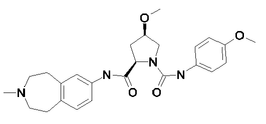 | 75% | (M+H)⁺ = 453 | R_{f}-Wert: 0.4 (Kieselgel; DCM/EtOH/NH3 = 80:20:2) |
| | (2R,4R)-4-Methoxy-pyrrolidin-1,2-dicarbonsäure-1-[(4-methoxyphenyl)-amid]-2-(3-methyl-2,3,4,5-tetrahydro-1*H*-benzo[*d*]azepin-7-yl)-amid | | | |
| **3** | 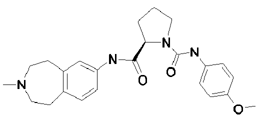 | 10% | (M+H)⁺ = 423 (M-H)⁻ = 421 | R_{f}-Wert: 0.56 (RP-8; MeOH/5%NaCl-Lsg. = 6:4) |
| | (2R)-Pyrrolidin-1,2-dicarbonsäure-1-[(4-methoxy-phenyl)-amid]-2-(3-methyl-2,3,4,5-tetrahydro-1*H*-benzo[*d*]azepin-7-yl)-amid | | | |
| **4** | 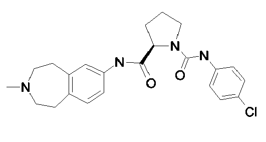 | 6% | (M+H)⁺ = 427/429 (Chlorisotope) | R_{f}-Wert: 0.44 (RP-8; MeOH/5%NaCl-Lsg. = 6:4) |
| | (2R)-Pyrrolidin-1,2-dicarbonsäure-1-[(4-chlor-phenyl)-amid]-2-(3-methyl-2,3,4,5-tetrahydro-1*H*-benzo[*d*]azepin-7-yl)-amid | | | |
| **5** | 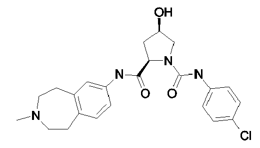 | 30% | (M+H)⁺ = 443/445 (Chlorisotope) | Rₜ-Zeit: 2.27 min (Methode A) |
| | (2R, 4R)-4-Hydroxy-pyrrolidin-1,2-dicarbonsäure-1-[(4-chlor-phenyl)-amid]-2-(3-methyl-2,3,4,5-tetrahydro-1*H*-benzo[*d*]azepin-7-yl)-amid | | | |
| **6** | 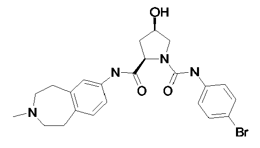 | 21% | (M+H)⁺ = 487/489 (Bromisotope) | Rₜ-Zeit: 2.28 min (Methode A) |
| | (2R,4R)-4-Hydroxy-pyrrolidin-1,2-dicarbonsäure-1-[(4-brom-phenyl)-amid]-2-(3-methyl-2,3,4,5-tetrahydro-1*H*-benzo[*d*]azepin-7-yl)-amid | | | |
| **7** | 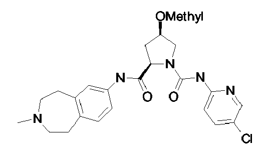 | 65% | (M+H)⁺ = 458/460 (Chlorisotope) | R_{f}-Wert: 0.3 (Kieselgel; DCM/EtOH/NH3 = 80:20:2) |
| | (2R,4R)-4-Methoxy-pyrrolidin-1,2-dicarbonsäure-1-[(5-chlor-pyridin-2-yl)-amid]-2-(3-methyl-2,3,4,5-tetrahydro-1*H*-benzo[*d*]azepin-7-yl)-amid | | | |
| **8** | 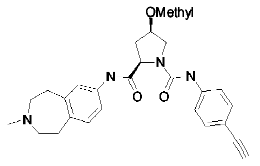 | 38% | (M+H)⁺ = 447 | R_{f}-Wert: 0.7 (Kieselgel; DCM/EtOH/NH3 = 80:20:2) |
| | (2R,4R)-4-Methoxy-pyrrolidin-1,2-dicarbonsäure-1-[(4-ethinyl-phenyl)-amid]-2-(3-methyl-2,3,4,5-tetrahydro-1*H*-benzo[*d*]azepin-7-yl)-amid | | | |
| **9** | 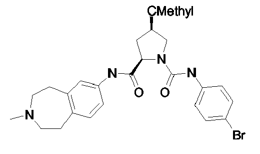 | 62% | (M+H)⁺ = 501/503 (Bromisotope) | Rₜ-Zeit: 4.17 min (Methode A) |
| | (2R,4R)-4-Methoxy-pyrrolidin-1,2-dicarbonsäure-1-[(4-brom-phenyl)-amid]-2-(3-methyl-2,3,4,5-tetrahydro-1*H*-benzo[*d*]azepin-7-yl)-amid | | | |
| **10** | 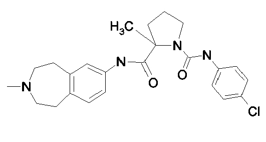 | 80% | (M+H)⁺ = 441/443 (Chlorisotope) | R_{f}-Wert: 0.6 (Kieselgel; DCM/EtOH/NH3 = 80:20:2) |
| | Pyrrolidin-2-methyl-1,2-dicarbonsäure-1-[(4-chlor-phenyl)-amid]-2-(3-methyl-2,3,4,5-tetrahydro-1*H*-benzo[*d*]azepin-7-yl)-amid | | | |

### Beispiel 11

### 2,5-Dihydro-pyrrole-1,2-dicarbonsäure-1-[(4-chlor-phenyl)-amid]-2-[(3-methyl-2,3,4,5-tetrahydro-1H-benzo[d]azepin-7-yl)-amid

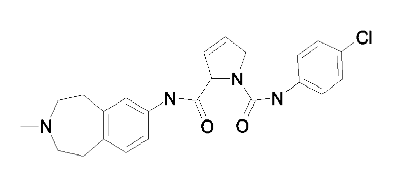

### (a) 1-(4-Chlor-phenylcarbamoyl)-2,5-dihydro-1H-pyrrol-2-carbonsäure

0.250 g (2.21 mmol) 3,4-Dehydro-DL-prolin werden in 15 ml 5%-NaHCO₃-Lösung gelöst, mit 0.678 ml (4.42 mmol) 4-Chlor-phenylisocyanat versetzt und 16 h bei 80°C gerührt. Anschließend wird abgekühlt, filtriert und der Rückstand mit Wasser gewaschen. Das Filtrat wird mit halbkonzentrierter HCl auf pH 1 gebracht, 2x mit Essigester extrahiert, mit Natriumsulfat getrocknet und i.Vak. konzentriert.
Ausbeute: 0.640 g (quantitativ, leicht unrein)
C₁₂H₁₁N₂O₃ (266.680)
Massenspektrum: (M+H)⁺ = 265/267 Chlorisotope

### (b) 2,5-Dihydro-pyrrole-1,2-dicarbonsäure-1-[(4-chlor-phenyl)-amid]-2-[(3-methyl-2,3,4,5-tetrahydro-1H-benzo[d]azepin-7-yl)-amid

0.200 g (0.750 mmol) 1-(4-Chlor-phenylcarbamoyl)-2,5-dihydro-1*H*-pyrrol-2-carbonsäure werden analog Beispiel 1a mit 0.132 g (0.750 mmol) 7-Amino-3-methyl-2,3,4,5-tetrahydro-1*H*-benzo[*d*]azepin, NMM und TBTU zur Titelverbindung umgesetzt.
Ausbeute: 50 mg (14%)
R_{f}-Wert: 0.6 (Kieselgel; Dichlormethan/Ethanol/Ammoniak = 80:20:2)
C₂₃H₂₅ClN₄O₂ (424.923)
Massenspektrum: (M+H)⁺ = 425/427 (Chlorisotope)

Analog können die folgenden Verbindungen hergestellt werden:

| **Bsp.** | **Strukturformel** | **Ausbeute** | **Massenpeak(s)** | **DC/HPLC** |
|---|---|---|---|---|
| **12** | 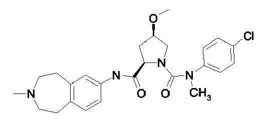 | | (M+H)⁺ = 471/473 (Chlorisotope) | |
| | (2R,4R)-4-Methoxy-pyrrolidin-1,2-dicarbonsäure-1-[N-(4-methoxy-phenyl)-N-methyl-amid]-2-(3-methyl-2,3,4,5-tetrahydro-1*H*-benzo[*d*]azepin-7-yl)-amid | | | |

Die nachfolgenden Beispiele beschreiben die Herstellung pharmazeutischer Anwendungsformen, die als Wirkstoff eine beliebige Verbindung der allgemeinen Formel I enthalten.

### Beispiel A

### Trockenampulle mit 75 mg Wirkstoff pro 10 ml

### Zusammensetzung:

| | | |
|---|---|---|
| Wirkstoff | 75.0 mg | |
| Mannitol | 50.0 mg | |
| Wasser für Injektionszwecke | | ad 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel B

### Trockenampulle mit 35 mg Wirkstoff pro 2 ml

### Zusammensetzung:

| | | |
|---|---|---|
| Wirkstoff | 35.0 mg | |
| Mannitol | 100.0 mg | |
| Wasser für Injektionszwecke | | ad 2.0 ml |

### Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

### Beispiel C

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel D

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

### Beispiel E

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel F

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel G

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Verbindungen der allgemeinen Formel (I) 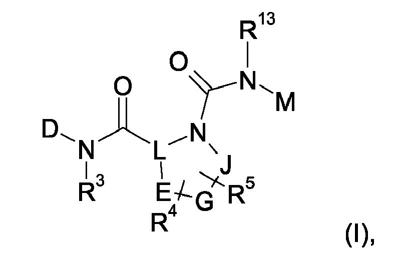 in denen
D ein substituiertes bicyclisches Ringsystem der Formel (II) 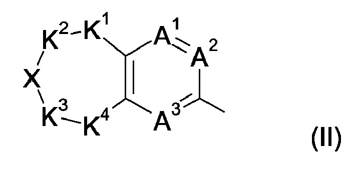 darstellt, in dem
K¹ und K⁴ jeweils unabhängig voneinander eine -CH₂-, -CHR^{7a}-, -CR^{7b}R^{7c}-oder eine -C(O)-Gruppe bedeuten, und wobei
R^{7a}/R^{7b}/R^{7c} jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe,
eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7a}/R^{7b}/R^{7c} zusammen mit dem Ringkohlenstoffatom oder den Ringkohlenstoffatomen einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon- oder [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem N-Atom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³ jeweils unabhängig voneinander eine -CH₂-, -CHR^{8a}-, -CR^{8b}R^{8c}- oder eine -C(O)-Gruppe bedeuten, wobei
R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem Stickstoffatom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt in Formel (II) maximal vier Reste ausgewählt aus R^{7a}, R^{7b}, R^{7c} R^{8a}, R^{8b} und R^{8c} vorhanden sein dürfen, und
X ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl-sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonyl-gruppe substituiert sein können, oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
-L-E-G-J- eine -C-C-C-C- oder -C-C=C-C-Gruppe bedeutet, und
R³ ein Wasserstoffatom oder eine C₁₋₃-Alkylgruppe bedeutet, und
R³ ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₀₋₃-alkyloxy-, Heteroaryl-C₀₋₃-alkyl-oxy-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom- und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet, und
wenn -L-E-G-J- eine -C-C-C-C-Gruppe bedeutet, kann R⁴ an E oder G auch ein Fluoratom oder eine Hydroxy-, Methoxy-, C₂₋₅-Alkenyl-oxy-, C₂₋₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₃Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom oder eine C₁₋₅Alkylgruppe bedeutet, oder sofern R⁵ mit E oder G verknüpft ist auch eine Hydroxy- oder Methoxy-gruppe darstellen kann, oder
R⁴ und R⁵ zusammen mit dem gleichen Kohlenstoffatom
eine -C(O)- Gruppe,oder eine -C(F₂)- Gruppe bilden können, oder
R⁴ und R⁵ zusammen mit dem gleichen Kohlenstoffatom oder zwei benachbarten Kohlenstoffatomen, an denen sie gebunden sind,
eine C₃₋₇-Cycloalkyl- oder C₅₋₇-Cycloalkenylgruppe bilden können,
wobei eine der Methylengruppen einer C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen einer C₄₋₇-Cycloalkylgruppe zusammen durch eine -C(O)NH-, - C(O)N(C₁₋₅-Alkyl)-, -S(O)₂NH-, oder -S(O)₂N(C₁₋₅-Alkyl)-Gruppe ersetzt sein können, und/oder
wobei 1 bis 3 Kohlenstoffatome einer C₃₋₇-Cycloalkylgruppe gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass eine solche, zusammen aus R⁴ und R⁵ gebildete C₃₋₇-Cycloalkylgruppe,
in der zwei Heteroatome im Cyclus aus der Gruppe Sauerstoff und Stickstoff durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
R¹³ ein Wasserstoffatom oder eine C₁₋₅Alkylgruppe bedeutet,
M einen gegebenenfalls durch R² und R⁶ substituierten Phenyl- oder Pyridylring bedeutet, in dem
R² ein Fluor-, Chlor-, Brom- oder Jod-atom oder eine Methyl-, Ethyl-, Vinyl-, Methoxy-, Ethinyl-, Cyano- oder -C(O)NH₂-Gruppe darstellt, und
R⁶ ein Wasserstoff-, Fluor-, Chlor-, Brom- oder Jod-atom oder eine Hydroxy-, Methoxy-, Trifluormethoxy-, eine gegebenenfalls durch Fluoratome substituierte C₁₋₃-Alkyl-, Cyano-, Amino-, oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

2. Verbindungen der allgemeinen Formel (I) nach Anspruch 1, in denen
D ein substituiertes bicyclisches Ringsystem der Formel (II) 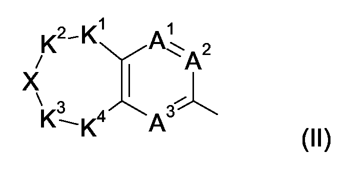 darstellt, in dem
K¹ und K⁴ jeweils unabhängig voneinander eine -CH₂-, -CHR^{7a}-, -CR^{7b}R^{7c}- oder eine -C(O)-Gruppe bedeuten, und wobei
R^{7a}/R^{7b}/R^{7c} jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer -CF₂-Gruppe,
K² und K³ jeweils unabhängig voneinander eine -CH₂-, -CHR^{8a}-, -CR^{8b}R^{8c}- oder eine -C(O)- Gruppe bedeuten, wobei
R^{8a}/R^{8b}/R^{8c} jeweils unabhängig voneinander eine C₁₋₅-Alkylgruppe bedeutet, und
insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten.

3. Verbindungen der allgemeinen Formel (I) nach Anspruch 1 oder 2, in denen
X eine NR¹-Gruppe bedeutet, in der
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten.

4. Verbindungen der allgemeinen Formel (I) nach einem der Ansprüche 1, 2 oder 3, in denen
R⁴ ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
wenn -L-E-G-J- eine -C-C-C-C-Gruppe bedeutet, kann R⁴ an E oder G auch ein Fluoratom oder eine Hydroxy-, Methoxy-, C₂₋₅-Alkenyl-oxy-, C₂₋₅-Alkinyl-oxy-, C₁₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₃Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom oder eine C₁₋₅Alkylgruppe bedeutet, oder sofern R⁵ mit E oder G verknüpft ist auch eine Hydroxy- oder Methoxy-gruppe darstellen kann, oder
R⁴ und R⁵ zusammen mit dem gleichen Kohlenstoffatom
eine -C(O)- Gruppe,oder eine -C(F₂)- Gruppe bilden können, oder
R⁴ und R⁵ zusammen mit dem gleichen Kohlenstoffatom oder zwei benachbarten Kohlenstoffatomen, an denen sie gebunden sind,
eine C₃₋₇-Cycloalkyl-gruppe bilden können,
wobei eine der Methylengruppen einer C₄₋₇-Cycloalkylgruppe durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Methylengruppen einer C₄₋₇-Cycloalkylgruppe zusammen durch eine -C(O)NH-, - C(O)N(C₁₋₅-Alkyl)-, -S(O)₂NH-, oder -S(O)₂N(C₁₋₅-Alkyl)-Gruppe ersetzt sein können.

5. Verbindungen der allgemeinen Formel (I) nach einem der Ansprüche 1, 2, 3 oder 4, in denen
-L-E-G-J- eine -C-C-C-C-Gruppe bedeutet.

6. Verbindungen der allgemeinen Formel (I) nach einem der Ansprüche 1, 2, 3, 4 oder 5, in denen
D einen substituierten Benzazepinylrest der Formel (IIa) 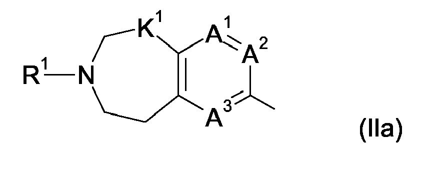 darstellt, in dem
K¹ eine -CH₂-, -CHR^{7a}-, -CR^{7b}R^{7c}- oder eine -C(O)-Gruppe bedeutet, wobei
R^{7a} eine C₁₋₅-Alkylgruppe bedeutet und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können, und
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
-L-E-G-J- eine -C-C-C-C-Gruppe bedeutet, und
R³ ein Wasserstoffatom bedeutet, und
R⁴ ein Wasserstoffatom oder
eine geradkettige oder verzweigte C₁₋₃-Alkylgruppe bedeutet, wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe bedeutet, oder
wenn R⁴ an E oder G angebunden ist auch ein Fluoratom oder eine Hydroxy-, Methoxy-, C₂₋₅-Alkenyl-oxy-, C₁₋₅-Alkyl-oxy-, C₃₋₆-Cycloalkyloxy-, C₁₋₅-Alkylaminocarbonyloxy-, Di(C₁₋₅-alkyl)aminocarbonyloxy- oder C₄₋₇-Cycloalkyleniminocarbonyloxy-gruppe darstellen kann,
R⁵ ein Wasserstoffatom oder eine C₁₋₅Alkylgruppe bedeutet, oder sofern R⁵ mit E oder G verknüpft ist auch eine Hydroxy- oder Methoxy-gruppe darstellen kann, oder
R⁴ und R⁵ zusammen mit dem Kohlenstoffatom an dem sie gebunden sind eine C=O- oder eine -CF₂-Gruppe bedeuten können, und
R¹³ ein Wasserstoffatom bedeutet,
M einen durch R² in 4-Position substituierten Phenyl- oder durch R² in 5-Position substituierten Pyridylring bedeutet, in dem
R² ein Fluor-, Chlor-, Bromatom, eine Methoxy- oder Ethinyl-Gruppe darstellt, und
R⁶ ein Wasserstoffatom darstellt.

7. Physiologisch verträgliche Salze der Verbindungen gemäß einem der Ansprüche 1 bis 6.

8. Arzneimittel, enthaltend eine Verbindung nach mindestens einem der Ansprüche 1 bis 6 oder ein physiologisch verträgliches Salz gemäß Anspruch 7, neben gegebenenfalls einem oder mehreren inerten Trägerstoffen und/oder Verdünnungsmitteln.

9. Verwendung einer Verbindung nach mindestens einem der Ansprüche 1 bis 6 oder eines physiologisch verträglichen Salzes gemäß Anspruch 7 zur Herstellung eines Arzneimittels mit einem inhibitorischen Effekt auf Faktor Xa und/oder einem inhibitorischen Effekt auf verwandte Serinproteasen.

10. Verfahren zur Herstellung eines Arzneimittels gemäß Anspruch 8, **dadurch gekennzeichnet, dass** auf nichtchemischem Wege eine Verbindung nach mindestens einem der Ansprüche 1 bis 6 oder ein physiologisch verträgliches Salz gemäß Anspruch 7 in einen oder mehrere inerte Trägerstoffe und/oder Verdünnungsmittel eingearbeitet wird.
